# EUROPEAN PATENT SPECIFICATION

(11) **EP 0 365 613 B1**
(45) Date of publication and mention of the grant of the patent: **17.05.1995**
(21) Application number: 89903311.2
(22) Date of filing: 09.03.1989
(51) Int. Cl.: A61K 31/74, A61K 31/78, A61K 31/71, A61K 31/35

(54) **COMBINATION OF TOBRAMYCIN AND STEROIDS FOR TOPICAL OPHTHALMIC USE**
KOMBINIERUNG VON TOBRAMYCIN UND STEROIDEN FÜR TOPISCHE OPHTHALMISCHE VERWENDUNG
TOBRAMYCINE ET STEROIDES COMBINES DESTINES A UNE UTILISATION OPHTALMIQUE TOPIQUE

(30) Priority: 09.03.1988 US 165950; 09.03.1988 US 165952
(43) Date of publication of application: 02.05.1990
(60) Divisional application: 93202516.6
(73) Proprietor: ALCON LABORATORIES, INC., Fort Worth Texas 76134-2099 (US)
(72) Inventor: CAGLE, Gerald, D., Fort Worth, TX 76133 (US); MCDONALD, Thomas, O., Fort Worth, TX 76107 (US); ROSENTHAL, Allan, L., Fort Worth, TX 76109 (US)
(74) Representative: Lederer, Franz, Dr.
(86) International application number: US8900952
(87) International publication number: WO8909057

(56) References cited:
- US-A- 4 407 792
- US-A- 4 474 753
- US-A- 4 478 822
- US-A- 4 668 506
- BIOLOGICAL ABSTRACTS, vol. 85, 1988, abstract no. 41064 R.D. SCHOENWALD et al.
- UNLISTED DRUGS, vol. 40, no. 11, Nov. 1988, page 218, abstract B: "TOBRADEX"
- BIOLOGICAL ABSTRACTS, abstract no. 86086941; R.S. STEWART et al.:"Efficacy and savety of tobramycin-dexamethasone ophthalmic suspention tobradex in prenvention of infection and reduction of inflammation following cataract surgery"

## Description

### Field of the Invention

This invention relates to the ophthalmic use of antibiotics in combination with anti-inflammatory steroids when indicated for treatment of ophthalmic infections and attendant inflammation.

### Background Art

Formulations of antibiotics or steroids are available in the ophthalmic art. However, there are concerns and expressed reservations in the ophthalmic community about the safety and efficacy of such prior art combinations. There is, moreover a long felt need for an effective and safe topical ophthalmic pharmaceutical composition of a potent steroid and a broad spectrum antibiotic which, when administered to the eye when indicated for bacterial infection or as a prophylactic after ophthalmic trauma and injury, will not, as a possible expression of the steroid component, inhibit the activity of the antibiotic or interfere with normal wound healing, but at the same time will control inflammation.

### Summary of the Invention

According to this invention it has been discovered that the broad spectrum aminoglycoside antibiotic tobramycin in combination with a potent steroid, dexamethasone, meet these criteria.

The present invention provides topical ophthalmic compositions which comprise the combination of the aminoglycoside antibiotic tobramycin with the potent steroid dexamethasone.

### Description of Preferred Embodiments

The present invention provides an antibiotic/antiinflammatory ophthalmic composition for topical delivery to the eye comprising a therapeutically effective amount of a mixture of the broad spectrum aminoglycoside antibiotic tobramycin in combination with a potent steroid dexamethasone, and a pharmaceutically acceptable carrier therefor. The amount of tobramycin and steroid will comprise a therapeutically effective amount of each substance in combination with a pharmaceutically acceptable carrier therefor. Preferably the ratio of tobramycin to steroid will range from 0.1 to 1.0 up to 10.0 to 1.0, respectively. Preferably, the mixture will contain about three times the weight of tobramycin to the weight of steroid.

Tobramycin is a water soluble, aminoglycoside antibiotic which is known chemically as 0-3 amino-3-deoxy-α-D-glucopyranosyl-(1→4)-0-[2,6-diamino-2,3,6-trideoxy-α-D-ribo-hexopyranosyl-(1→6)]-2-deoxy-L-streptamine. Tobramycin is reported as compound 9318 in the Merck Index, 10th Ed., 1983.

Dexamethasone is a synthetic analog of cortisol and possesses a high anti-inflammatory potency. It has a molecular weight of 392.4 and its chemical name is 9-fluoro-11β,17,21-trihydroxy-16α-methylpregna-1,4-diene-3,20-dione. Dexamethasone is disclosed as compound 2906 in the Merck Index, 10 ED., 1983.

The compositions of the present invention comprise mixtures of the tobramycin and steroid in the amounts indicated. The composition will also contain other ingredients conventionally used in ophthalmic preparations such as antimicrobial preservatives, co-solvents and viscosity agents.

The compositions of the present invention are administered topically to humans and animals. The dosage range is 0.001 to 5.0 mg/per eye, wherein the cited mass figures represent the sum of the two components, dexamethasone and tobramycin. The compositions of the present invention can be administered as solutions, suspensions, or emulsions (dispersions) in a suitable ophthalmic vehicle.

In forming compositions for topical administration, the mixtures of antibiotic and steroid are preferably formulated as 0.01 to 2.0 percent by weight solutions in water at a pH of 4.5 to 8.0 (figures relate to combined presence of tobramycin and steroid). While the precise regimen is left to the discretion of the clinician, it is recommended that the resulting solution be topically applied by placing one drop in each eye of the human or animal two times a day.

### Antimicrobial Preservative:

Ophthalmic products are typically packaged in multidose form. Preservatives are thus required to prevent microbial contamination during use. Suitable preservatives include: benzalkonium chloride, thimerosal, chlorobutanol, methyl paraben, propyl paraben, phenylethyl alcohol, edetate disodium sorbic acid, Onamer M, or other agents known to those skilled in the art. Typically such preservatives are employed at a level of from 0.001% to 1.0% by weight in the formulation.

### Co-Solvents:

The solubility of the components of the present compositions may be enhanced by a surfactant or other appropriate co-solvent in the composition. Such co-solvents include polysorbate 20, 60, and 80, Pluronic® F-68, F-84 and P-103, cyclodextrin, or other agents known to those skilled in the art. Typically such co-solvents are employed at a level of from 0.01% to 2% by weight in the formulation.

### Viscosity Agents:

Viscosity increased above that of simple aqueous solutions may be desirable to increase ocular absorption of the active compound, to decrease variability in dispensing the formulation, to decrease physical separation of components of a suspension or emulsion of the formulation and/or to otherwise improve the ophthalmic formulation. Such viscosity builder agents include as examples polyvinyl alcohol, polyvinyl pyrrolidone, methyl cellulose, hydroxy propyl methycellulose, hydroxyethyl cellulose, carboxymethyl cellulose, hydroxy propyl cellulose or other agents known to those skilled in the art. Such agents are typically employed at a level of from 0.01% to 2% by weight.

The combination of tobramycin and dexamethasone of the present invention provides a topical agent which takes advantage of the broad spectrum of activity of the tobramycin for ocular pathogens including some strains resistant to other antibiotics because it has a low potential for development of resistance and a lower incidence of sensitivity reactions than other antibiotics such as neomycin which are often used in steroid combinations.

In the combination pharmaceutical composition of this invention, it has been found that the tobramycin remains a highly effective antibiotic against susceptible strains of microorganisms such as staphylococci including s. aureus and s.epidermidis, including penicillin resistant strains, as well as streptococci including some of the group A species and some streptococcus pneumoniae. The combination pharmaceutical composition has been found effective in treating most external ocular infections caused by bacteria.

The compositions of the present invention are directed to the treatment of patients (i.e., "hosts") who exhibit the symptoms associated with ophthalmic infections and attendant inflammation, as well as to the treatment of inflammation in patients who may be predisposed to developing an ophthalmic infection, either as a result of an immunosuppressant effect attributable to the steroid component of the composition or as the result of a condition (e.g., injury) unrelated to the steroid therapy. The symptoms associated with these conditions are well known to ophthalmologists (i.e., medical doctors specializing in the treatment of eye disorders), as well as other physicians and clinicians having expertise in the treatment of ophthalmic infections and inflammation.

There has been a long felt need in the ophthalmic field for an improved, effective and safe combination of an antibiotic and an antiinflammatory steroid. In order to be both effective and safe, one component cannot interfere with or alter the action of the other component. For example, the steroid component must be capable of controlling inflammation without interfering with the action of the antibiotic. The present invention is based on the discovery that a particular combination, tobramycin and dexamethasone satisfies these criteria.

The toxicology of the combination pharmaceutical composition of the invention has been shown to have a margin of safety equal to or greater than that found with other antibiotic-steroid combinations. Further, clinical efficacy studies found that the combination pharmaceutical compositions are safe and effective when used prior to and after ocular surgery.

As indicated above the composition of the present invention are particularly for use as ophthalmic compositions to be applied topically, preferably by administration of drops in the eye as clinically indicated. The preferred composition comprises a composition which contains 0.3 weight percent of tobramycin and 0.1 weight percent of the steroid, dexamethasone. The mixtures are formed by conventional mixing of the required amounts of each of the active materials in combination with the other components desired to be included in the composition.

The following examples are representative pharmaceutical compositions of the invention for topical use when indicated against inflammation and infection. Parts are by weight unless otherwise indicated. The examples set forth preferred formulations.

### EXAMPLE I

| | | |
|---|---|---|
| Dexamethasone, Micronized USP | 1.0 mg + 5% excess | 0.10% + 5% excess |
| Tobramycin, USP | 3.0 mg + 5% excess | 0.30% + 5% excess |
| Benzalkonium Chloride Solution (10%), NF | 0.001 ml+10% excess | 0.10%+10% excess¹ |
| Edetate Disodium, USP | 0.1 mg | 0.01% |
| Sodium Chloride, USP | 3.0 mg | 0.3% |
| Sodium Sulfate, USP | 12.0 mg | 1.2% |
| Tyloxapol, USP | 0.5 mg | 0.05% |
| Hydroxyethylcellulose | 2.5 mg | 0.25% |
| Sulfuric Acid and/or Sodium Hydroxide, NF | QS for pH adjustment to 5.5 ± 0.5 | |
| Purified Water, USP | QS to 1 ml | QS to 100% |

| | | |
|---|---|---|
| ¹The benzalkonium chloride, NF concentration is equivalent to 0.01% (+ 10% excess). | | |

### EXAMPLE II

| | | |
|---|---|---|
| Dexamethasone, Micronized, USP | 0.1% + 2% excess | 1 mg + 2% excess |
| Tobramycin, Micronized, USP | 0.3% + 7% excess | 3 mg + 7% excess |
| Chlorobutanol, Anhydrous, NF | 0.5% + 25% excess | 5 mg + 15% excess |
| Mineral Oil, USP | 5% | 50 mg |
| White Petrolatum, USP | QS 100% | QS 1 g |

### EXAMPLE V

### Clinical Efficacy

A controlled multi-center study was carried out with three investigators to ascertain the safety and efficacy of the ophthalmic composition of this invention relative to MAXITROL® ophthalmic suspension in the prevention of post-operative infection and in the control of post-operative inflammation after cataract surgery. The ophthalmic suspension of this invention was the formulation of Example 1 which contained .3 wt% of tobramycin and .1 wt% of dexamethasone. MAXITROL® is a commercially available multi-dose anti-infective steroid combination in sterile suspension for topical application. The active ingredient is dexamethasone in 0.1 wt% amounts. The other active ingredients are neomycin sulfate and Polymyxin sulfate. MAXITROL® is indicated for steroid responsive inflammatory ocular conditions for which a corticoid steroid is indicated and where bacterial infection or a risk of bacterial ocular infection exists. See Physician's Desk Reference for Ophthalmology, 12 Ed., 1984, p, 72. These studies were double masked and randomized. After the pressure enrollment examination, each patient was given a coded medication and instructed to dose one drop every four hours at home for three days prior to surgery. Post-surgical examinations were performed at days 1, 4, 7, 14 and 21. Dosing was as follows: 2 drops every 2 hours while awake for 2 days beginning with the first ocular dressing change the day after surgery; one drop four times per day for the next 7 days; 1 one drop daily for the next 10 days.

Of the 73 patients evaluated for efficacy, 36 patients received the composition of Example I and 37 were dosed with MAXITROL®. The two treatment groups did not differ in demographic characteristics, initial signs and symptoms, type of cataract, concomitant systemic medications and type of extraction. Surgically induced changes of the bulbur conjunctiva, palpervaral conjectiva and limbus were reported along with the following signs: aqueous reaction, erythema, epithelial disease, discharge, exudation, focal stromal infiltrates, iris damage and vitreous reaction. The results demonstrated no significant differences in the signs presented after surgery or in the rate of resolution of those signs.

Ocular infections did not occur in either treatment group. The effectiveness of the composition of this invention in controlling post-surgical inflammation after cataract surgery indicated effectiveness parallel to that of MAXITROL® for the control of post-surgical inflammation and demonstrated parallel effectiveness with regard to prophylaxis of post-operative infection and control of post-operative inflammation. Further, the composition of the invention was safe and effective when used prior to and after ocular surgery.

The invention has been described herein by reference to certain preferred embodiments.

## Claims

1. An antibiotic/antiinflamatory ophthalmic pharmaceutical composition for topical delivery to the eye comprising a therapeutically effective amount of a mixture of tobramycin and the steroid dexamethasone, and a pharmaceutically acceptable carrier therefor.

2. An antibiotic/antiinflammatory ophthalmic pharmaceutical composition according to claim 1 wherein the ratio of tobramycin to steroid is in the range of from 0.1:1.0 to 10.0:1.0.

3. A composition according to claim 1 in the form of an aqueous solution, suspension, or emulsion containing 0.01 to 2.0 wt. % of the composition at a pH of 4.5 to 8.0.

4. A composition according to claim 3 which contains 0.3 wt. % tobramycin and 0.1 wt. % dexamethasone.

5. A composition according to claim 1 which also contains one or more preservatives, co-solvents, and/or viscosity building agents.

6. Use of a therapeutically effective amount of a mixture of tobramycin and the steroid dexamethasone, for the manufacture of a combined antibiotic and antiinflammatory ophthalmic composition for topical delivery to the eye for treatment of inflammation and/or bacterial infection and/or as a prophylactic after ophthalmic trauma or injury.

7. Use according to claim 6, wherein the ratio of tobramycin to steroid is in the range of from 0.1:1.0 to 10.0:1.0.

8. Use according to claim 6, wherein the composition is in the form of an aqueous solution, suspension, or emulsion containing 0.01 to 2.0 wt. % of the combined mixture of tobramycin and steroid at a pH of 4.5 to 8.0.

9. Use according to claim 8 which contains 0.3 wt. % tobramycin and 0.1 wt. % steroid.

## Patentansprüche

1. Eine antibiotisch/entzündungshemmende, ophthalmologische, pharmazeutische Zusammensetzung zur topischen Aufbringung auf das Auge, enthaltend eine therapeutisch wirksame Menge einer Mischung von Tobramycin und dem Steroid Dexamethason und einem pharmazeutisch verträglichen Träger hierfür.

2. Eine antibiotische/entzündungshemmende, ophthalmologische, pharmazeutische Zusammensetzung nach Anspruch 1, **dadurch gekennzeichnet**, daß das Verhältnis von Tobramycin zu dem Steroid im Bereich zwischen 0,1:1,0 und 10,0:1,0 liegt.

3. Eine Zusammensetzung nach Anspruch 1 in Form einer wäßrigen Lösung, Suspension oder Emulsion, enthaltend 0,01 bis 2,0 Gewichtsprozent der Zusammensetzung bei einem pH-Wert von 4,5 bis 8,0.

4. Eine Zusammensetzung nach Anspruch 3, die 0,3 Gewichtsprozent Tobramycin und 0,1 Gewichtsprozent Dexamethason enthält.

5. Eine Zusammensetzung nach Anspruch 1, die ebenfalls ein oder mehrere Konservierungsmittel, Co-Lösungsmittel und/oder Mittel zur Erhöhung der Viskosität enthält.

6. Die Verwendung einer therapeutisch wirksamen Menge einer Mischung von Tobramycin und dem Steroid Dexamethason zur Herstellung einer kombinierten, antibiotischen und entzündungshemmenden, ophthalmologischen Zusammensetzung zur topischen Aufbringung auf das Auge zur Behandlung von Entzündungen und/oder bakteriellen Infektionen und/oder als ein Prophylaktikum nach einem ophthalmologischen Trauma oder einer Augenverletzung.

7. Verwendung nach Anspruch 6,
**dadurch gekennzeichnet**,
daß das Verhältnis von Tobramycin zu dem Steroid im Bereich zwischen 0,1:1 und 10,0:1,0 liegt.

8. Verwendung nach Anspruch 6,
**dadurch gekennzeichnet**,
daß die Zusammensetzung in Form einer wäßrigen Lösung, Suspension oder Emulsion vorliegt, die 0,01 bis 2,0 Gewichtsprozent der kombinierten Mischung von Tobramycin und dem Steroid bei einem pH-Wert von 4,5 bis 8,0 enthält.

9. Verwendung nach Anspruch 8,
**dadurch gekennzeichnet**,
daß die Zusammensetzung 0,3 Gewichtsprozent Tobramycin und 0,1 Gewichtsprozent Steroid enthält.

## Revendications

1. Composition pharmaceutique ophtalmique antibiotique/anti-inflammatoire pour délivrance topique à l'oeil, comprenant une quantité efficace du point de vue thérapeutique d'un mélange de tobramycine et du stéroïde dexaméthasone, et un véhicule, acceptable du point de vue pharmaceutique, pour ceux-ci.

2. Composition pharmaceutique ophtalmique antibiotique/anti-inflammatoire selon la revendication 1, dans laquelle le rapport de la quantité de tobramycine sur la quantité de stéroïde est compris dans l'intervalle de 0,1:1,0 à 10,0:1,0.

3. Composition selon la revendication 1, sous forme d'une solution, d'une suspension ou d'une émulsion aqueuse contenant de 0,01 à 2,0 % en poids de la composition à un pH compris entre 4,5 et 8,0.

4. Composition selon la revendication 3, qui contient 0,3 % en poids de tobramycine et 0,1 % en poids de dexaméthasone.

5. Composition selon la revendication 1, qui contient également un ou plusieurs conservateurs, cosolvants, et/ou agents donnant de la viscosité.

6. Utilisation d'une quantité efficace du point de vue thérapeutique d'un mélange de tobramycine et du stéroïde dexaméthasone, pour la fabrication d'une composition ophtalmique antibiotique et anti-inflammatoire combinée pour délivrance topique à l'oeil, pour le traitement d'inflammation et/ou d'infection bactérienne et/ou en tant que produit prophylactique après un traumatisme ou une blessure ophtalmique.

7. Utilisation selon la revendication 6, dans lequel le rapport de la quantité de tobramycine sur la quantité de stéroïde est compris dans l'intervalle de 0,1:1,0 à 10,0:1,0.

8. Utilisation selon la revendication 6, dans lequel la composition est sous forme d'une solution, d'une suspension ou d'une émulsion aqueuse contenant de 0,01 à 2,0 % en poids du mélange combiné de tobramycine et de stéroïde à un pH compris entre 4,5 et 8,0.

9. Utilisation selon la revendication 8, qui contient 0,3 % en poids de tobramycine et 0,1 % en poids de stéroïde.
